(19) Europäisches Patentamt
European Patent Office
Office européen des brevets

(11) Publication number: **0 172 383 B1**

(12) **EUROPEAN PATENT SPECIFICATION**

(45) Date of publication of patent specification: **05.02.92**

(51) Int. Cl.[5]: **C07D 473/08**, C07D 295/10

(21) Application number: **85108634.8**

(22) Date of filing: **11.07.85**

The file contains technical information submitted after the application was filed and not included in this specification

(54) **Pharmacologically active salt derivatives of 1,2,3,6-tetrahydro-1,3-dimethyl-2,6 dioxopurine-7-acetic acid.**

(30) Priority: **06.08.84 IT 2223584**

(43) Date of publication of application:
**26.02.86 Bulletin 86/09**

(45) Publication of the grant of the patent:
**05.02.92 Bulletin 92/06**

(84) Designated Contracting States:
**AT CH DE FR LI**

(56) References cited:
**CH-A- 373 759**
**GB-A- 2 004 883**

**Lespagnol, Chimie des médicaments, T.2, pp. 8, 419-21 (1974)**

(73) Proprietor: **LABORATORIO ITALIANO BIOCHIMICO FARMACEUTICO LISAPHARMA S.P.A.**
**Via Licinio 11, 13, 15**
**I-22036 Erba (Como)(IT)**

(72) Inventor: **Zagnoli, Giorgio**
**via Rubini 7**
**Como(IT)**

(74) Representative: **Gervasi, Gemma, Dr. et al**
**Studio Brevetti e Marchi NOTARBARTOLO & GERVASI 33, Viale Bianca Maria**
**I-20122 Milano(IT)**

EP 0 172 383 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new pharmacologically active salt derivative, and precisely to 4-(1-pyrrolidinyl)-1-(2,4,6-trimethoxyphenyl)-1-butanone 1,2,3,6-tetrahydro-1,3-dimethyl-2,6-dioxopurine-7-acetate, having the following structural formula:

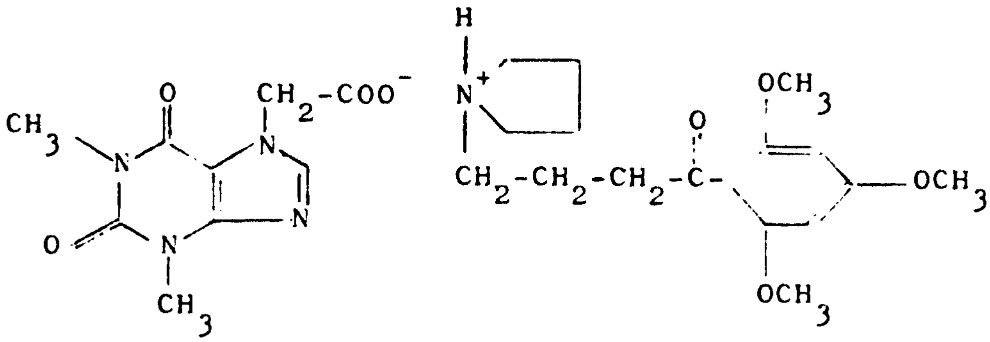

(1)

The present invention relates moreover to the process for the production of the salt derivative (I) and to the related pharmaceutical compositions.

The salt derivative (I) is obtained by means of the salification of 1,2,3,6-tetrahydro-1,3-dimethyl-2,6-dioxopurine-7-acetic acid with 4-(1-pirrolidinyl)-1-(2,4,6-trimethoxyphenyl)-1-butanone.

The pharmacological activity of the two starting products is known.

It is known in particular that 1,2,3,6-tetrahydro-1,3-dimethyl-2,6-dioxopurine-7-acetic acid (theophyllineacetic acid) exerts a coronary vasodilator and stimulating action on the cardial circulation, and the pharmacological activity is further known of 4-(1-pyrrolidinyl)-1-(2,4,6-trimethoxy-phenyl)-1-butanone hydrochloride (Buflomedil) on the cerebral circulation, for the treatment of the consciousness diseases, of states consequent to cerebral traumata, and moreover its peripheral vasodilator property.

The salt derivative (I) according to the present invention has, in the clinical field, an important application in the treatment of senility, thanks to its stimulating activity of the respiratory function, to its peripheral vasodilator activity, to its diuretic, antithrombotic, cardial stimulating and coronarodilator activity.

All these activities are associated in the salt derivative (I) according to the present invention, which displays moreover a high water solubility, which favours its bioavailability and allows its administration also by the parenteral and rectal way.

The salt derivative (I) according to the present invention is prepared by means of a process characterized in that 1,2,3,6-tetrahydro-1,3-dimethyl-2,6-dioxopurine-7-acetic acid is reacted with 4-(1-pyrrolidinyl)-1-(2,4,6-trimethoxy-phenyl)-1-butanone in a reaction medium constituted by an organic solvent wherein (I) is highly soluble, and that the reaction product (I) is crystallized from an organic solvent wherein it has a low solubility.

The characteristics and advantages of the salt derivative (I) and of the related production process shall be better evidenced by the following disclosure, which is referred to a preferred embodiment.

For the preparation of the salt derivative (I), 1,2,3,6-tetrahydro-1,3-dimethyl-2,6-dioxopurine-7-acetic acid is dissolved in an organic solvent, and the solution is heated at a temperature comprised within the range of from 40 °C to the boiling temperature of the solvent, and, preferably, of from 45 to 55 °C.

The solvent is preferably an alifatic alcohol containing from 1 to 4 carbon atoms.

To the so-obtained alcoholic solution, 4-(1-pyrrolidinyl)-1-(2,4,6-trimethoxy-phenyl)-1-butanone is added, a complete solution being obtained. The two reactants, which are present in the stoichiometric molar ratio, or in a ratio close to the stoichiometric one, give rise to a reaction in solution.

The solution is cooled and concentrated in vacuo and to the residue ethyl acetate anhydrous and preheated to a temperature comprised within the range of from 50 to 70 °C is then added.

By cooling at a temperature comprised within the range of from 0 to 25 °C, the salt derivative (I) crystallizes, which is filtered, washed with ethyl acetate and then dried in vacuo at 35 - 45 °C. The yield of the raw product is of about 80 - 85% of the theoretical value.

The salt derivative (I) obtained has the appearance of a white crystalline powder, very soluble in water, alifatic alcohols and chloroform, whilst it is insoluble in ethyl acetate.

The crystallization of the raw product is carried out from a mixture of alifatic alcohols and ethyl acetate, until the required purity degree is obtained. The yield of each crystallization is of about 90%.

The characterization of said product is carried out by: X-ray diffractometry, I.R, U.V. and N.M.R. spectrography and by means of the determination of the melting temperature and of pH.

The crystallographic analysis by X-ray diffractometry shows that the product is a single substance deriving from the formation of a ionic bond between the two reactants.

In the following Table 1 the characteristic peaks (diffraction angles $\theta$ and interplanar distances of $\lambda_{Cu}$ = 1.5418 Å and the intensity on relative scale $I_{rel}$, for the salt derivative (I) according to the invention, and, to comparative purposes, for the two reactants (Buflomedil and theophyllineacetic acid) are reported.

TABLE 1

| | | $2\theta_{Cu}$ | d | $I_{rel}$ |
|---|---|---|---|---|
| Buflomedil | | | | |
| | a | 19.7 | 4.51 | (5.3) |
| | b | 23.9 | 3.72 | (4.6) |
| | c | 15.9 | 5.57 | (3.9) |
| | d | 21.2 | 4.19 | (3.9) |
| | e | 16.3 | 5.44 | (3.4) |
| Theophyllineacetic acid | | | | |
| | a | 11.9 | 7.44 | (7.3) |
| | b | 24.2 | 3.68 | (3.4) |
| | c | 16.0 | 5.54 | (2.6) |
| | d | 12.3 | 7.20 | (1.9) |
| | e | 10.4 | 8.51 | (1.8) |
| Salt derivative (I) | | | | |
| | a | 22.5 | 3.95 | (5.2) |
| | b | 22.9 | 3.88 | (3.4) |
| | c | 21.8 | 4.08 | (3.2) |
| | d | 18.7 | 4.75 | (2.5) |
| | e | 16.8 | 5.28 | (2.3) |

It results from this table than no one of the characteristic peaks of the reactants is present in the salt derivative (I) and vice-versa; in the salification of the reactants a new crystalline phase has been hence obtained. The U.V. spectrum shows an absorption maximum at 275 ± 1 nm.

The melting temperature, as determined on Kofler block, is well defined at the value of 108 - 111 °C. pH, as determined in an aqueous solution at 2%, is of 7.4.

As for the pharmaceutical compositions in which the salt derivative (I) according to the invention may enter as the active principle, it may be observed that, thanks to its high solubility in water, with it all pharmaceutical forms can be prepared, both for oral and rectal administering, and for the injectable or topic way, in association with the usual conventional pharmaceutical excipients and media.

To illustrative, but not limitative purpose of the process according to the present invention, the following Example is reported.

Example 1

Fourty grams of 1,2,3,6-tetrahydro-1,3-dimetyl-2,6-dioxopurine-7-aceticacid ($C_9H_{10}N_4O_4$, M.W. = 238.2) are dissolved in 100 ml of absolute ethanol, and the solution is heated at 50° C.

In it, 51.7 g of 4-(pyrrolidinyl)-1-(2,4,6-trimethoxy-phenyl)-1-butanone ($C_{17}H_{25}NO_4$,M.W. = 307) are introduced.

The solution obtained is cooled, is concentrated in vacuo to a low volume, and is poured into 450 ml of anhydrous ethyl acetate preheated at 60° C.

The salt then precipitates by cooling to a temperature comprised within the range of from 0° to 25° C, and is filtered, washed with ethyl acetate and dried in vacuo at 40° C. Seventy-six grams of 4-(1-pyrrolidinyl)-1-(2,4,6-trimethoxyphenyl)-1-butanone 1,2,3,6-tetrahydro-1,3-dimethyl-2,6-dioxopurine-7-acetate ($C_{26}H_{35}N_5O_8$, M.W. = 545.2) are obtained, having after two crystallizations from absolute ethanol / ethyl acetate 1 : 2 the following chemical-physical characteristics:
Melting point: 108 - 111° C
U.V. absorption maximum at 275 ± 1 nm
Titer in 1,2,3,6-tetrahydro-2,3-dimethyl-2,6-dioxopurine-7-acetic acid: 42.92
Titer in N-4-(pyrrolidinyl)-1-(2,4,6-trimethoxyphenyl)-1-butanone: 57.08
pH of a 10% aqueous solution: 7.4.

**Claims**
**Claims for the following Contracting States : CH, DE, FR, LI**

1. Pharmacologically active salt derivative constituted by 4-(1-pyrrolidinyl)-1-(2,4,6-trimethoxyphenyl)-1-butanone 1,2,3,6-tetrahydro-1,3-dimethyl-2,6-dioxopurine-7-acetate, having the following structural formula:

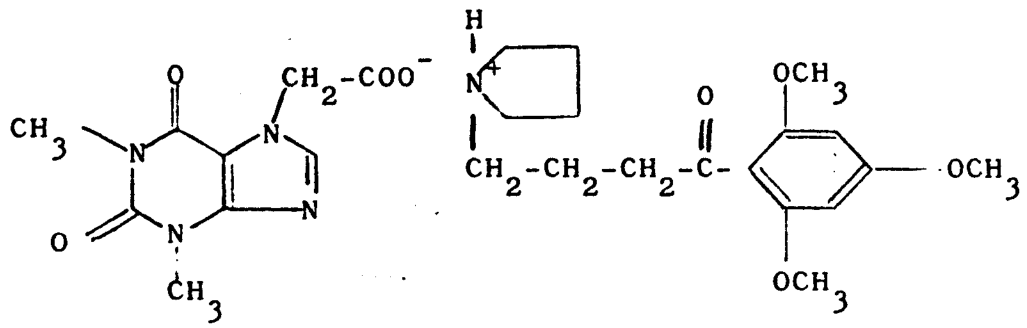

(I)

2. Process for the preparation of the pharmacologically active salt derivative according to claim 1 characterized in that 1,2,3,6-tetrahydro-1,3-dimethyl-2,5-dioxopurine-7-acetic acid is reacted with 4-(1-pirrolidinyl)-1-(2,4,6 -trimethoxyphenyl)-1-butanone in a reaction medium constituted by an organic solvent, wherein (I) is highly soluble, and that the reaction product (I) is crystallized from an organic solvent, wherein it is poorly soluble.

3. Process according to claim 2, characterized in that said reaction is carried out at a temperature comprised within the range of from 40° C to the boiling temperature of the solvent under refluxing conditions, and preferably at a temperature comprised within the range of from 45 to 55° C.

4. Process according to claim 2, characterized in that said reaction medium is constituted by an alifatic alcohol containing from 1 to 4 carbon atoms.

5. Process according to claim 2, characterized in that the reactants are reacted in an about stoichiometric molecular ratio.

6. Process according to claim 2, characterized in that said crystallization solvent is constituted by ethyl acetate or by mixtures of alifatic alcohols and ethyl acetate.

7. Process according to claim 2, characterized in that said crystallization of the reaction product (I) from the solvent constituted by ethyl acetate or by mixtures of alifatic alcohols and ethyl acetate is carried out by cooling at a temperature comprised within the range of from 0° to 25° C.

**Claims for the following Contracting State : AT**

1. Process for the preparation of the pharmacologically active salt derivative constituted by 4-(1-pirrolidinyl)-1-(2,4,6-trimethoxyphenyl)-1-butanone 1,2,3,6-tetrahydro-1,3-dimethyl-2,6-dioxopurine-7-acetate, having the following structural formula:

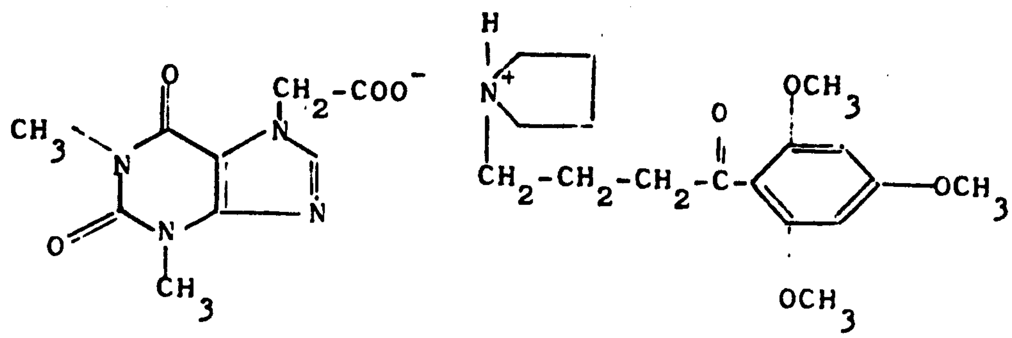

characterized in that 1,2,3,6-tetrahydro-1,3-dimethyl-2,5-dioxopurine-7-acetic acid is reacted with 4-(1-pirrolidinyl) -1-(2,4,6 -trimethoxyphenyl)-1-butanone in a reaction medium constituted by an organic solvent, wherein (I) is highly soluble, and that the reaction product (I) is crystallized from an organic solvent, wherein it is poorly soluble.

2. Process according to claim 1, characterized in that said reaction is carried out at a temperature comprised within the range of from 40° C to the boiling temperature of the solvent under refluxing conditions, and preferably at a temperature comprised within the range of from 45 to 55° C.

3. Process according to claim 1, characterized in that said reaction medium is constituted by an alifatic alcohol containing from 1 to 4 carbon atoms.

4. Process according to claim 1, characterized in that the reactants are reacted in an about stoichiometric molecular ratio.

5. Process according to claim 1, characterized in that said crystallization solvent is constituted by ethyl acetate or by mixtures of alifatic alcohols and ethyl acetate.

6. Process according to claim 1, characterized in that said crystallization of the reaction product (I) from the solvent constituted by ethyl acetate or by mixtures of alifatic alchols and ethyl acetate is carried out by cooling at a temperature comprised within the range of from 0° to 25° C.

**Revendications**
**Revendications pour les Etats contractants suivants : CH, DE, FR, LI**

1. Dérivé de sel pharmacologiquement actif consitué par le 4-(1-pyrrolidinyl)-1-(2,4,6-triméthoxyphényl)-1-butanone 1,2,3,6-tétrahydro-1,3-diméthyl-2,6-dioxopurine-7-acétate, ayant la formule structurelle suivante :

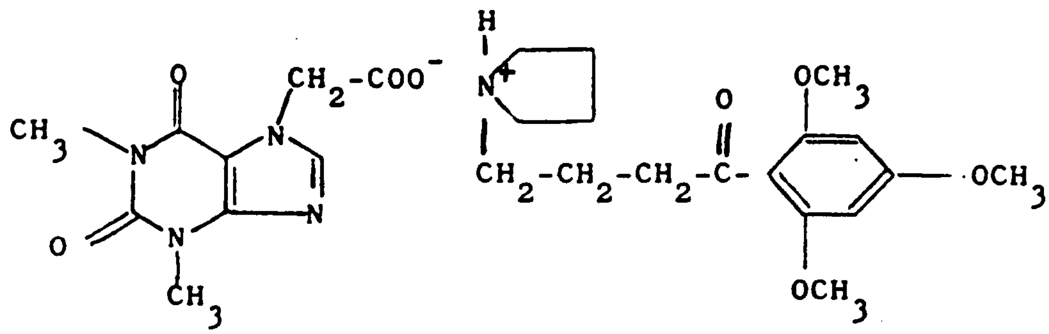

(I)

2. Procédé de préparation du dérivé de sel pharmacologiquement actif selon la revendication 1, caractérisé en ce que l'on fait réagir l'acide 1,2,3,6-tétrahydro-1,3-diméthyl-2,5-dioxopurine-7-acétique avec la 4-(1-pyrrolidinyl)-1-(2,4,6-triméthoxyphényl)-1-butanone dans un milieu réactionnel constitué par un solvant organique, dans lequel (I) est extrêmement soluble, et en ce que le produit de la réaction (I) est cristallisé à partir d'un solvant organique dans lequel il est faiblement soluble.

3. Procédé selon la revendication 2, caractérisé par le fait que ladite réaction est effectuée à une température comprise à l'intérieur de la plage allant de 40 C° jusqu'à la température d'ébullition du solvant dans des conditions de reflux, et de préférence à une température comprise à l'intérieur de la plage allant de 45 C° à 55 C°.

4. Procédé selon la revendication 2, caractérisé par le fait que ledit milieu réactionnel est constitué par un alcool aliphatique contenant de 1 à 4 atomes de carbone.

5. Procédé selon la revendication 2, caractérisé par le fait que les corps réagissant sont mis en réaction dans un rapport moléculaire sensiblement égal au rapport stoechiométrique.

6. Procédé selon la revendication 2, caractérisé par le fait que le solvant de cristallisation est constitué par l'acétate d'éthyle ou par des mélanges d'alcools aliphatiques et d'acétate d'éthyle.

7. Procédé selon la revendication 2, caractérisé par le fait que la cristallisation du produit de la réaction (I) à partir du solvant constitué par l'acétate d'éthyle ou par des mélanges d'alcools aliphatiques et d'acétate d'éthyle est effectuée par refroidissement à une température comprise à l'intérieur de la plage allant de 0 C° à 25 C°.

**Revendications pour l'Etat contractant suivant : AT**

1. Procédé de préparation du dérivé de sel pharmacologiquement actif consitué par le 4-(1-pyrrolidinyl)-1-(2,4,6-triméthoxyphényl)-1-butanone 1,2,3,6-tétrahydro-1,3-diméthyl-2,6-dioxopurine-7-acétate, ayant la formule structurelle suivante :

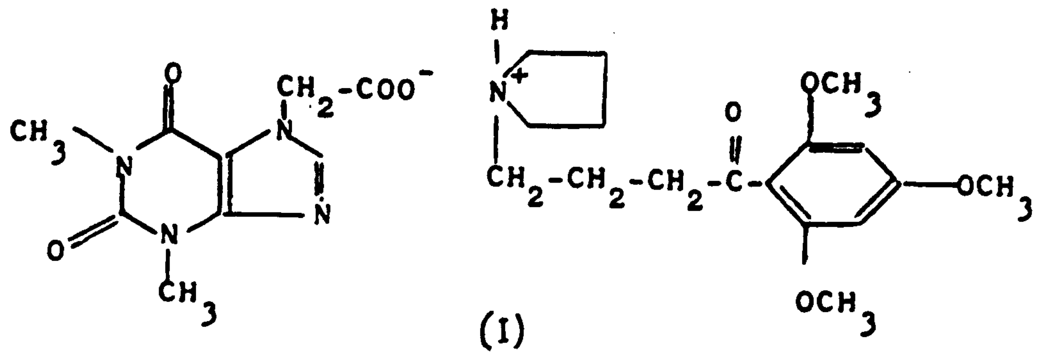

(I)

caractérisé en ce que l'on fait réagir l'acide 1,2,3,6-tétrahydro-1,3-diméthyl-2,5-dioxopurine-7-acétique avec la 4-(1-pyrrolidinyl)-1-(2,4,6-triméthoxyphényl)-1-butanone dans un milieu réactionnel constitué par un solvant organique, dans lequel (I) est extrêmement soluble, et en ce que le produit de la réaction (I) est cristallisé à partir d'un solvant organique dans lequel il est faiblement soluble.

2. Procédé selon la revendication 1, caractérisé par le fait que ladite réaction est effectuée à une température comprise à l'intérieur de la plage allant de 40 C° jusqu'à la température d'ébullition du solvant dans des conditions de reflux, et de préférence à une température comprise à l'intérieur de la plage allant de 45 C° à 55 C°.

3. Procédé selon la revendication 1, caractérisé par le fait que ledit milieu réactionnel est constitué par un alcool aliphatique contenant de 1 à 4 atomes de carbone.

4. Procédé selon la revendication 1, caractérisé par le fait que les corps réagissant sont mis en réaction dans un rapport moléculaire sensiblement égal au rapport stoechiométrique.

5. Procédé selon la revendication 1, caractérisé par le fait que le solvant de cristallisation est constitué par l'acétate d'éthyle ou par des mélanges d'alcools aliphatiques et d'acétate d'éthyle.

6. Procédé selon la revendication 1, caractérisé par le fait que la cristallisation du produit de la réaction (I) à partir du solvant constitué par l'acétate d'éthyle ou par des mélanges d'alcools aliphatiques et d'acétate d'éthyle est effectuée par refroidissement à une température comprise à l'intérieur de la plage allant de 0 C° à 25 C°.

**Patentansprüche**

**Patentansprüche für folgende Vertragsstaaten : CH, DE, FR, LI**

1. Pharmakologisch aktives Salzderivat, gebildet von 4-(1-Pyrrolidinyl)-1-(2,4,6-trimethoxyphenyl)-1-butanon-1,2,3,6-Tetrahydro-1,3-dimethyl-2,6-dioxopurin-7-acetat der folgenden Strukturformel (I):

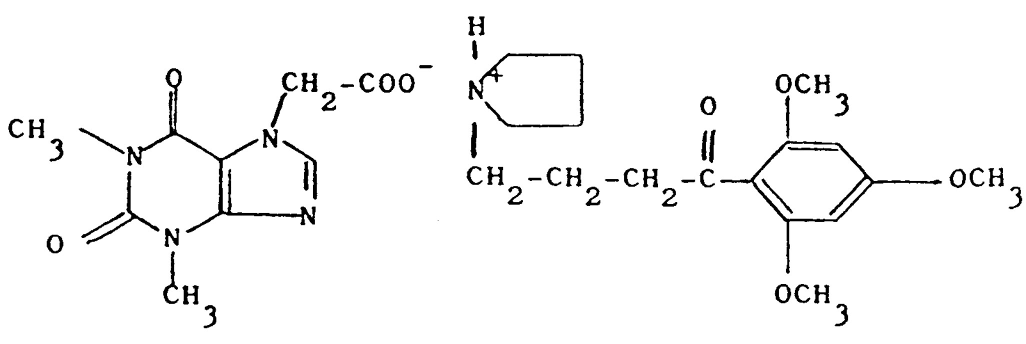

(I)

2. Verfahren zur Herstellung des pharmakologisch aktiven Salzderivat nach Anspruch 1, dadurch gekennzeichnet, daß 1,2,3,6-Tetrahydro-1,3-dimethyl-2,6-dioxopurin-7-essigsäure mit 4-(1-Pyrrolidinyl)-1-(2,4,6-trimethoxyphenyl)-1-butanon in einem durch ein organisches Lösungsmittel gebildeten Reaktionsmedium, in dem (I) leicht löslich ist, umgesetzt wird, und daß das Reaktionsprodukt (I) aus einem organischen Lösungsmittel, in dem es schwer löslich ist, kristallisiert wird.

3. Verfahren nach Anspruch 2, dadurch gekennzeichnet, daß die Umsetzung bei einer Temperatur im Bereich von 40° C bis zum Siedepunkt des Lösungsmittels unter Rückflußbedingungen, und vorzugsweise bei einer Temperatur im Bereich von 45 bis 55° C durchgeführt wird.

4. Verfahren nach Anspruch 2, dadurch gekennzeichnet, daß das Reaktionsmedium durch einen 1 bis 4 Kohlenstoffatome enthaltenden aliphatischen Alkohol gebildet wird.

5. Verfahren nach Anspruch 2, dadurch gekennzeichnet, daß die Reaktanten in einem etwa stöchiometrischen Molverhältnis umgesetzt werden.

6. Verfahren nach Anspruch 2, dadurch gekennzeichnet, daß das Kristallisationslösungsmittel durch Ethylacetat oder durch Mischungen von aliphatischen Alkoholen und Ethylacetat gebildet wird.

7. Verfahren nach Anspruch 2, dadurch gekennzeichnet, daß die Kristallisation des Reaktionsproduktes (I) aus dem durch Ethylacetat oder durch Mischungen von aliphatischen Alkoholen und Ethylacetat gebildeten Lösungsmittel durch Abkühlen bei einer Temperatur im Bereich von 0° bis 25° C durchgeführt wird.

**Patentansprüche für folgenden Vertragsstaat : AT**

1. Verfahren zur Herstellung des pharmakologisch aktiven Salzderivats, gebildet von 4-(1-Pyrrolidinyl)-1-(2,4,6-trimethoxyphenyl)-1-butanon-1,2,3,6-Tetrahydro-1,3-dimethyl-2,6-dioxopurin-7-acetat der folgenden Strukturformel (I):

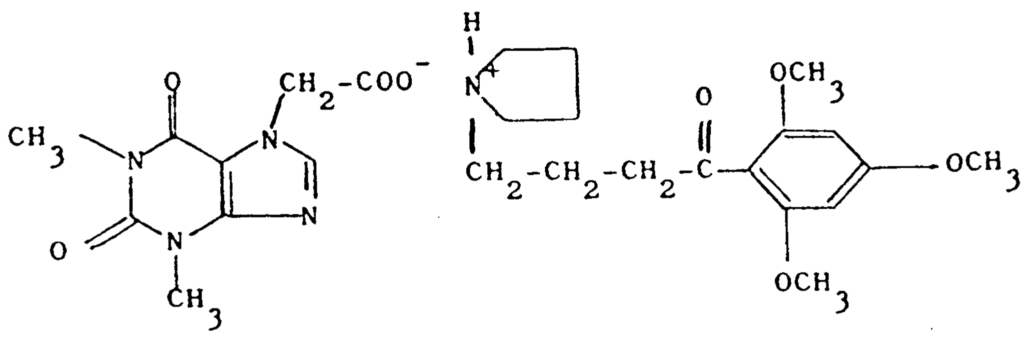 (I),

dadurch gekennzeichnet, daß 1,2,3,6-Tetrahydro-1,3-dimethyl-2,6-dioxopurin-7-essigsäure mit 4-(1-Pyrrolidinyl)-1-(2,4,6-trimethoxyphenyl)-1-butanon in einem durch ein organisches Lösungsmittel gebildeten Reaktionsmedium, in dem (I) leicht löslich ist, umgesetzt wird, und daß das Reaktionsprodukt (I) aus einem organischen Lösungsmittel, in dem es schwer löslich ist, kristallisiert wird.

2. Verfahren nach Anspruch 1, dadurch gekennzeichnet, daß die Umsetzung bei einer Temperatur im Bereich von 40° C bis zum Siedepunkt des Lösungsmittels unter Rückflußbedingungen, und vorzugsweise bei einer Temperatur im Bereich von 45 bis 55° C durchgeführt wird.

3. Verfahren nach Anspruch 1, dadurch gekennzeichnet, daß das Reaktionsmedium durch einen 1 bis 4 Kohlenstoffatome enthaltenden aliphatischen Alkohol gebildet wird.

4. Verfahren nach Anspruch 1, dadurch gekennzeichnet, daß die Reaktanten in einem etwa stöchiometrischen Molverhältnis umgesetzt werden.

5. Verfahren nach Anspruch 1, dadurch gekennzeichnet, daß das Kristallisationslösungsmittel durch Ethylacetat oder durch Mischungen von aliphatischen Alkoholen und Ethylacetat gebildet wird.

**6.** Verfahren nach Anspruch 1, dadurch gekennzeichnet, daß die Kristallisation des Reaktionsproduktes (I) aus dem durch Ethylacetat oder durch Mischungen von aliphatischen Alkoholen und Ethylacetat gebildeten Lösungsmittel durch Abkühlen bei einer Temperatur im Bereich von 0° bis 25°C durchgeführt wird.